# EUROPÄISCHE PATENTANMELDUNG

(11) **EP 1 872 771 A2**
(43) Veröffentlichungstag der Anmeldung: **02.01.2008**
(21) Anmeldenummer: 07110583.7
(22) Anmeldetag: 19.06.2007
(51) Int. Cl.: A61K 8/41, A61K 8/46, A61K 8/73, A61K 8/81, A61Q 5/02, A61Q 5/12, A61Q 19/10

(54) **Haarpflegereinigungszusammensetzung mit besonderer Trockenhaarsensorik**

(30) Priorität: 28.06.2006 DE 102006030083
(71) Anmelder: Beiersdorf AG, 20253 Hamburg (DE)
(72) Erfinder: Aechtner, Anja, 68165, Mannheim (DE); Albrecht, Harald, 8320, Fehraltorf (CH); Heitmann, Birgit, 22769, Hamburg (DE); Neuhoff, Henrike, 22359, Hamburg (DE); Wilken, Maren, 22848, Norderstedt (DE); Ruppert, Stephan, 20259, Hamburg (DE)

(57) **Zusammenfassung**

Kosmetische und dermatologische waschaktive Zubereitungen zur Reinigung der Haut und/oder zur Haar- und Kopfhautreinigung, enthaltend Dimethyldiallylammoniumchlorid-Acrylamid Copolymere, kationisch modifizierte Guarderivate, Alkylethersulfate und Cocamidopropylbetain.

## Beschreibung

Die vorliegende Erfindung betrifft kosmetische Reinigungsmittel mit besonderer Pflegeleistung. Kosmetische Reinigungsmittel sind an sich bekannt. Es handelt sich dabei im wesentlichen um oberflächenaktive Substanzen oder Stoffgemische, die dem Verbraucher in verschiedenen Zubereitungen angeboten werden.

Unter Pflegeleistung im Sinne der vorliegenden Schrift versteht man das Vermögen der Zusammensetzung beim Benutzer derselben sauberes Haar, eine gute Kämmbarkeit des Haares, im nassen und trockenen Zustand, eine gute Frisierbarkeit, einen guten Griff und einen guten Haarglanz zu erzielen.

Zubereitungen dieser Art sind beispielsweise Schaum- und Duschbäder, Shampoos, spezielle Reinigungsmittel für Kleinkinder und Babys und dergleichen.

Moderne Haarreinigungsprodukte bestehen im Allgemeinen aus Wasser, Tensiden, Verdicker, Hilfsstoffen und Pflegekomponenten.

Besonders den Pflegekomponenten kommt in neuerer Zeit eine hohe Bedeutung zu, da der Verbraucher, speziell jener mit strapaziertem/geschädigtem Haar, eine sehr hohe Pflegeleistung erwartet. Pflegende- bzw. konditionierende Substanzen sind Stoffe, die eine hohe Affinität zum Haar haben und auf die Haaroberfläche aufziehen wobei sie das Haar möglichst nicht beschweren. Sie glätten so die Haarstruktur und erleichtern dadurch das Kämmen der Haare. Gleichzeitig verhindern sie die elektrostatische Aufladung und verleihen dem Haar Glanz. Eine entscheidende Rolle bei den Konditionierstoffen kommt dabei auch der Sensorik - dem Gefühl und Griff den sie im nassen und trockenen Haar hervorrufen, zu. Pflege ist daher gleichbedeutend mit den biophysikalischen Parametern Kämmbarkeit, Volumen, Fülle, Glanz, Griff und Geschmeidigkeit.

Pflegekomponenten werden generell in zwei Gruppen aufgeteilt: wasserlösliche und wasserunlösliche Konditionierstoffe. Zu den wasserunlöslichen Konditionierstoffen gehören Silikone, Öle, Fettalkohole und Wachse; zu den Wasserlöslichen kationische Tenside und Kationische Polymere.

Kationische Polymere sind für Shampoo-Formulierungen essentielle Rohstoffe. Zur Erzielung eines kontrollierten Konditioniereffektes ist es heutzutage unumgänglich, das richtige Polymer mit der geeigneten Konzentration einzusetzen. Eine einfache Erhöhung der Polymereinsatzkonzentration führt leider oft zu so genannten "build up - Effekten", wobei das Polymer verstärkt auf das Haar aufzieht und nur schwer wieder abgewaschen kann. Das Haar wird dadurch schwer, belegt und schmierig.

Wünschenswert ist es daher, ein maßgeschneidertes Polymersystem einzusetzen, dass hohe Pflegeeigenschaften hat und das Haar nicht beschwert.

Untersuchungen haben gezeigt, dass verschiedene kationische Polymere durchaus unterschiedliche Effekte am Haar bewirken. Polyquaternium-44 ist beispielsweise ein effektiver Wirkstoff, der in Kombination mit einer entsprechenden Tensidbasis (LES; Coco-Glucosid) die Frisierbarkeit und den Frisurenhalt signifikant verbessert (W. Seipel, P. Hössel, N. Boyxen, W. Eisfeld, Polymere und geeignete Wirkstoffe für die Entwicklung von Shampoo-Formulierungen; SÖFW-Journal, 2003, 129. J., 69-76.).

Die Performance von Haarpflegeprodukten im nassen und insbesondere auch im trockenen Haar ist für den Verbraucher spürbar nachvollziehbar und somit von großer Bedeutung. Entsprechend ist die Auswahl der kationischen Polymere und des Tensidsystems in einer Shampooformulierung für die Sensorik im nassen und trockenen Haar maßgeblich entscheidend. Insbesondere die Sensorik im trockenen Haar, die Geschmeidigkeit und der Griff spielen für den Verbraucher eine wichtige Rolle, da diese Parameter nach der Shampooapplikation bis zur nächsten Anwendung anhaltend erlebt werden.

Es ist daher vorteilhaft, den Verbrauchern eine auf die oben genannten Parameter gut abgestimmte Shampooformulierung anzubieten. Üblicherweise werden in Shampooformulierungen kationische Polymere in Kombination mit Silikonen eingesetzt, um eine verstärkte Ablagerung der Silikone auf dem Haar und somit eine hohe Pflegeleistung und gute Kämmbarkeit zu erzielen. Silikone haben sehr gute konditionierende Eigenschaften, bilden aber oft einen Film auf dem Haar (EP 0 432 951 und EP 0 529 883). In der WO 98/0424 wird ein Konditionersystem bestehend aus unlöslichen Silikonölen und Polyolcarbonsäureestern und entsprechende Mischungen davon eingesetzt.

Oft werden auch Polyolcarbonsäureester in Kombination mit kationischen Zellulosederivaten eingesetzt (WO 98/04241). In der WO 93/08787 wird ein Konditioniersystem bestehend aus 3 Komponenten beschrieben (ein unlösliches Silikon, ein kationisches Polymer mit bestimmter Ladungsdichte und ein flüssiges Öl). In der EP 1 158 952 B1 wird ein Konditioniersystem bestehend aus einem unlöslichen Silikon (z. B.: Polydimetylsiloxan oder einem Silikon mit einer Aminogruppe) in Kombination mit einem Fettsäureester und einem kationischen Polymer geschützt.

Andere konditionierende Haarwaschmittelformulierungen beschreiben die Verwendung von anionischen Tensiden (insbesondere Alkylethersulfaten bzw. deren Salze in Kombination mit Carboxlaten wie Alkylpolyethercarbonsäuren bzw. deren Salze, quaternären Ammoniumalkylamiden) mit kationischen Polymeren wie z. B. Celluloseetherderivate vom Typ "Polymer JR" oder Diallyldimethylammoniumchlorid-Derivate vom Typ "Merquat" und quaternisierten Phosphatestern (DE 19937916 A1; DE 19937917 A; WO 9710804A1; DE 10309180A1) zur Erzielung einer guten Reinigungsleistung und Schleimhautverträglichkeit.

Wünschenswert ist dagegen, dem Verbraucher nicht nur herkömmliche Shampoos mit guter Reinigungsleistung anzubieten, sondern zusätzlich einen spürbaren Benefit mitzugeben. Herkömmliche Shampoos mit Silikonen und kationischen Polymeren machen das Haar oft belegt. Daher wäre die Entwicklung eines reinigenden Shampoos, das zugleich das Haar spürbar pflegt und insbesondere dem trockenen Haar eine ausgezeichnete Geschmeidigkeit gibt, erstrebenswert.

Auch die Haarwäsche mit aggressiven Tensiden kann das Haar beanspruchen, zumindest dessen Erscheinungsbild oder das Erscheinungsbild der Haartracht insgesamt herabsetzen. Beispielsweise können bestimmte wasserlösliche Haarbestandteile (z.B. Harnstoff, Harnsäure, Xanthin, Keratin, Glycogen, Citronensäure, Milchsäure) durch die Haarwäsche herausgelaugt werden.

Der Stande der Technik ließ es aber an Shampooformulierungen mangeln, welche dem geschädigten Haar in befriedigender Weise Pflege zukommen ließen. Aufgabe war daher, auch diesen Nachteilen des Standes der Technik Abhilfe zu schaffen.

Es hat sich überraschend gezeigt, und darin liegt die Lösung dieser Aufgaben, dass kosmetische und dermatologische waschaktive Zubereitungen zur Reinigung der Haut und/oder zur Haar- und Kopfhautreinigung, enthaltend Dimethyldiallylammoniumchlorid-Acrylamid Copolymere, kationisch modifizierte Guarderivate, Alkylethersulfate und Cocamidopropylbetain. den Nachteilen des Standes der Technik abhelfen. Es wurde, was für den Fachmann völlig überraschend war, eine konditionierende Haarreinigungsformulierung entwickelt, die insbesondere die Sensorik im trockenen Haar, den Griff und die Geschmeidigkeit, gegenüber herkömmlichen Formeln entscheidend und für den Verbraucher nachvollziehbar, verbessert. Dabei ist es bevorzugt, wenn zusätzlich Alkylpolyglykolestersulfosuccinate enthalten sind. Erfindungsgemäß vorteilhaft ist es, wenn ein Dimethyldiallylammoniumchlorid-Acrylamid Copolymer mit einem Molekulargewicht von 1,0x10⁶ g/mol bis 3x10⁶ g/mol und einer Ladungsdichte von 1,0 bis 3,5 meg/g verwendet wird, bevorzugt mit einem Molekulargewicht von 1,4x10⁶ g/mol bis 1,8x10⁶ g/mol und einer Ladungsdichte von 2,5 bis 3,5 meg/g, besonders bevorzugt mit einem Molekulargewicht von 1,6x10⁶ g/mol und einer Ladungsdichte von 3,1 meg/g. Weiter bevorzugt ist es, wenn als kationisch modifizierte Guarderivate solche mit einem Molekulargewicht von 50.000 bis 2.500.000, besonders bevorzugt von 1.000.000 bis 1.500.000 verwendet werden. Besonders bevorzugt ist es, wenn als kationisch modifizierte Guarderivate solche mit einem Molekulargewicht von 1.300000 g/mol und einer mittleren Ladungsdichte von 0.4-0.6 meq/g verwendet werden. Ganz besonders bevorzugt ist es, wenn als kationisch modifiziertes Guarderivat Guar Hydroxypropyltrimonium Chlorid verwendet wird. Weiter bevorzugt ist es, wenn die Einsatzkonzentrationen an Dimethyldiallylammoniumchlorid-Acrylamid Copolymer und kationisch modifizierten Guarderivaten jeweils 0,01 bis 1 Gew.-%, besonders bevorzugt 0,01 bis 0,5 Gew.-% betragen. Besonders geeignet war Dimethyldiallylammoniumchlorid-Acrylamid Copolymeren vom Typ "Merquat" in Kombination mit kationisch modifizierten Guarderivaten, insbesondere Guar Hydroxypropyltrimoniumchlorid sowie Alkylethersulfat und Cocamidopropylbetain als Tensidsystem. Bei der Kombination von Dimethyldiallylammoniumchlorid-Acrylamid Copolymer und Guar Excel haben sich Einsatzkonzentrationen von jeweils 0,01 - 1 % als vorteilhaft gezeigt. Besonders geeignet waren Einsatzkonzentrationen von 0,01 - 0,5 %.

Weiter bevorzugt ist es, wenn mindestens ein Di-Natrium Salz eines Alkylpolyglycolestersulfosuccinats der Äpfel-, Milch- oder Citronensäure enthalten ist. Weiter bevorzugt ist es, wenn als Tenside anionische, amphotere und nichtionische Tenside in Gesamtkonzentrationen von 2 bis 25 Gew.-% verwendet werden. Weiter bevorzugt ist es, wenn als Tenside die Kombination von Laurylethersulfat mit Cocamidopropylbetain verwendet wird. Besonders vorteilhaft ist Laurylethersulfat in Konzentrationen von 5-15 Gew.-% und Cocamidopropylbetain in Konzentration von 0.5 - 10 Gew.-% einzusetzen. Ganz besonders vorteilhaft ist es Laurylethersulfat in Konzentrationen von 6-12 Gew.-% und Cocamidopropylbetain in Konzentrationen von 1 - 5 Gew.-% einzusetzen.

Als Verdicker werden bevorzugt Natriumchlorid, PEG-200 hydrogeniertes Glycerylpalmitat und Acrylat Copolymere verwendet. Besonders vorteilhaft sind Einsatzkonzentrationen von 0.05 - 5 Gew.-%.

Weiter bevorzugt ist es, wenn zusätzlich PEG-3 Distearat mit Teilchengrößen verwendet wird, die so bemessen sind, dass 90% der Volumenanteile eine Teilchengröße von kleiner 50µm aufweisen. Die Einsatzkonzentrationen des Perlglanzes liegen vorteilhafterweise bei 5 - 15 Gew.-%.

Anstelle des Perlglanzsystems kann auch ein Trübungsmittel eingesetzt werden. Besonders vorteilhaft ist der Einsatz von Mischungen, bestehend aus Glycol Distearat, Coco-Glucosid, Glyceryl Oleat, Glyceryl Stearate wie sie unter dem Handelsnamen Lamesoft TM Benz (Cognis) angeboten weden.

Vorteilhaft kann auch eine Kombination von Perlglanz und Trübungsmittel eingesetzt werden.

Anionische Tenside im Sinne der Erfindung weisen als funktionelle Gruppen in der Regel Carboxylat-, Sulfat- oder Sulfonatgruppen auf. In wässriger Lösung bilden sie im sauren oder neutralen Milieu negativ geladene organische Ionen. Kationische Tenside sind beinahe ausschließlich durch das Vorhandensein einer quarternären Ammoniumgruppe gekennzeichnet. In wässriger Lösung bilden sie im sauren oder neutralen Milieu positiv geladene organische lonen. Amphotere Tenside enthalten sowohl anionische als auch kationische Gruppen und verhalten sich demnach in wässriger Lösung je nach pH-Wert wie anionische oder kationische Tenside. Im stark sauren Milieu besitzen sie eine positive und im alkalischen Milieu eine negative Ladung. Im neutralen pH-Bereich hingegen sind sie zwitterionisch, wie das folgende Beispiel verdeutlichen soll:

RNH₂⁺CH₂CH₂COOH X⁻ (bei pH=2) X⁻ = beliebiges Anion, z.B. Cl⁻

RNH₂⁺CH₂CH₂COO⁻ (bei pH=7)

RNHCH₂CH₂COO⁻ B⁺ (bei pH=12) B⁺ = beliebiges Kation, z.B. Na⁺

Typisch für nicht-ionische Tenside sind Polyether-Ketten. Nicht-ionische Tenside bilden in wässrigem Medium keine Ionen.
A. Anionische Tenside
   Erfindungsgemäß vorteilhaft zu verwendende anionische Tenside sind Acylaminosäuren (und deren Salze), wie
   1. Acylglutamate, beispielsweise Natriumacylglutamat, Di-TEA-palmitoylaspartat und Natrium Caprylic/ Capric Glutamat,
   2. Acylpeptide, beispielsweise Palmitoyl-hydrolysiertes Milchprotein, Natrium Cocoyl-hydrolysiertes Soja Protein und Natrium-/ Kalium-Cocoyl-hydrolysiertes Kollagen,
   3. Sarcosinate, beispielsweise Myristoyl Sarcosin, TEA-lauroyl Sarcosinat, Natriumlauroylsarcosinat und Natriumcocoylsarkosinat,
   4. Taurate, beispielsweise Natriumlauroyltaurat und Natriummethylcocoyltaurat,
   5. Acyllactylate, Lauroyllactylat, Caproyllactylat
   6. Alaninate
   Carbonsäuren und Derivate, wie
   1. Carbonsäuren, beispielsweise Laurinsäure, Aluminiumstearat, Magnesiumalkanolat und Zinkundecylenat,
   2. Ester-Carbonsäuren, beispielsweise Calciumstearoyllactylat, Laureth-6-Citrat und Natrium PEG-4-Lauramidcarboxylat,
   3. Ether-Carbonsäuren, beispielsweise Natriumlaureth-13-Carboxylat und Natrium PEG-6-Cocamide Carboxylat,
   Phosphorsäureester und Salze, wie beispielsweise DEA-Oleth-10-Phosphat und Dilaureth-4 Phosphat,
   Sulfonsäuren und Salze, wie
   1. Acyl-isethionate, z.B. Natrium-/ Ammoniumcocoyl-isethionat,
   2. Alkylarylsulfonate,
   3. Alkylsulfonate, beispielsweise Natriumcocosmonoglyceridsulfat, Natrium C₁₂₋₁₄ Olefinsulfonat, Natriumlaurylsulfoacetat und Magnesium PEG-3 Cocamidsulfat,
   4. Sulfosuccinate, beispielsweise Dioctylnatriumsulfosuccinat, Dinatriumlaurethsulfosuccinat, Dinatriumlaurylsulfosuccinat und Dinatriumundecylenamido-MEA-Sulfosuccinat
   sowie
   Schwefelsäureester, wie
   1. Alkylethersulfat, beispielsweise Natrium-, Ammonium-, Magnesium-, MIPA-, TIPA-Laurethsulfat, Natriummyrethsulfat und Natrium C₁₂₋₁₃-Parethsulfat,
   2. Alkylsulfate, beispielsweise Natrium-, Ammonium- und TEA-Laurylsulfat.
B. Amphotere Tenside
   Erfindungsgemäß vorteilhaft zu verwendende amphotere Tenside sind
   1. Acyl-/dialkylethylendiamin, beispielsweise Natriumacylamphoacetat, Dinatriumacylamphodipropionat, Dinatriumalkylamphodiacetat, Natriumacylamphohydroxypropylsulfonat, Dinatriumacylamphodiacetat und Natriumacylamphopropionat,
   2. N-Alkylaminosäuren, beispielsweise Aminopropylalkylglutamid, Alkylaminopropionsäure, Natriumalkylimidodipropionat und Lauroamphocarboxyglycinat.

Vorteilhafte nichtionische Tenside mit einem HLB-Wert von 10 bis 20 können beispielsweise sein:
PEG-20 Mandelölglyzerid, PEG-20 Nachtkerzenölglyzerid, PEG-60 hydrogeniertes Rizinusöl, Polysorbat 60, PEG-20 Sorbitan Isostearat, PEG-40 hydrogeniertes Rizinusöl, Polysorbat 80, Succrose Cocoat, PEG-45 Palmkernölglyzeride, PEG-45 Distelölglyzerid, PEG-60 Nachtkerzenölglyzerid, PEG-42 Babassuölglyzerid, PEG-20 Rizinusoleat, Steareth-21, Oleth-20, Polysorbat 40, Laureth-9, Laureth-15, Ceteareth-20, Ceteth-20, PEG-12 Laurat, PEG-24 Stearat, PEG-20 Stearat, PEG-20 Oleat, PEG-75 Lanolin, Laneth-40, PEG-8 Dilaurat, Polysorbat 65, PEG-7 Glyceryl Cocoat, Laneth-10, PEG-12 Oleat, Polyglyzeryl-6 Laurat, Polyglyzeryl-10 Laurat, Polyglyzeryl-10 Myristat, Polyglyzeryl-10 Stearat, Polyglyzeryl-10 Oleat, Polyglyzeryl-10 Isostearat, Polyglyzeryl-10 Diisostearat, PEG-15 Glyzeryloleat, PEG-60 Sorbitan Tetraoleat, PEG-10 Oleylether

Die erfindungsgemäßen waschaktiven Zubereitungen zeichnen sich in der Regel durch einen Wassergehalt von 95-5 Gew.-% aus, bezogen auf das Gesamtgewicht der Zubereitungen und stellen Gele dar.

Zubereitungen gemäß der Erfindung sind vorteilhaft auf einen pH-Bereich > 4,2 gepuffert, besonders bevorzugt > 4,5 besonders bevorzugt 4,8-7,5.

Die Herstellung der erfindungsgemäßen kosmetischen und/oder dermatologischen Zubereitungen erfolgt in der dem Fachmann üblichen Weise, zumeist dergestalt, dass die erfindungsgemäß verwendeten grenzflächenaktiven Stoffe bei gleichmäßigem Rühren und gegebenenfalls unter Erwärmen suspendiert und gewünschtenfalls homogenisiert, gegebenenfalls mit weiteren Lipidkomponenten und gegebenenfalls mit einem oder mehreren weiteren Emulgatoren vereinigt, hernach die Ölphase mit der wässrigen Phase, in welche gegebenenfalls ein Verdickungsmittel eingearbeitet worden ist, und welche vorzugsweise etwa die gleiche Temperatur besitzt wie die Ölphase, vermischt, gewünschtenfalls homogenisiert und auf Raumtemperatur abkühlen lässt. Nach Abkühlen auf Raumtemperatur kann, insbesondere, wenn noch flüchtige Bestandteile eingearbeitet werden sollen, nochmaliges Homogenisieren erfolgen.

### Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

Guar Hydroxypropyltrimonium Chlorid und PQ-7 werden mit einem Teil der Wasserphase verdünnt und unter Rühren zur Tensidphase gegeben. Anschließend werden die weiteren Rezepturbestandteile unter Rühren zugegeben und mit NaOH der pH-Wert eingestellt. Bei all dem wird unter möglichst geringer Scherung gerührt.

### Shampoos

### 1.) Conditioner-Shampoo

| | **1** | **2** | **3** | **4** | **5** | **6** | **7** |
|---|---|---|---|---|---|---|---|
| Natrium Laurylethersulfat | 7 | 7 | 8 | 9 | 9 | 10 | 12 |
| Cocamidopropyl Betain | - | 3 | 3.5 | 3 | 4 | 4 | 3 |
| Di-Natrium PEG-5 Laurylcitrat Sulfosuccinat | - | - | 2 | 3 | 3 | - | - |
| Decyl Glucoside | - | 2 | 3 | - | - | - | - |
| Natrium Cocoamphoacetat | 3 | - | - | - | - | - | - |
| Guar Hydroxypropyltrimonium Chloride | 0.1 | 0.2 | 0.2 | 0.3 | 0.1 | 0.4 | 0.5 |
| Polyquaternium-7 | 0.5 | 0.1 | 0.1 | 0.2 | 0.3 | 0.1 | 0.2 |
| PEG-200 Hydrogenated Glyceryl Palmate | - | - | 0,2 | - | - | 1,0 | - |
| PEG-40 hydrogeniertes Rizinusöl | 0.2 | 0.3 | 0.4 | 0,5 | 0.6 | 0,5 | 0.5 |
| PEG-3 Distearat | 1.5 | - | 0.75 | 1.5 | 1.0 | - | 0.5 |
| Glycol Distearat | - | 4.0 | - | - | - | - | - |
| PEG-90M | - | - | - | 0.05 | - | 0.1 | 0.2 |
| PEG-7 Glycerylcocoat | 0.5 | 1 | 2 | - | - | - | - |
| Natrium Salicylat | 0.2 | 0.3 | 0.4 | 0.4 | 0.4 | - | - |
| Natrium Benzoat | 0.5 | 0.4 | 0.3 | 0.2 | 0.4 | - | 0.5 |
| Parfüm | q.s. | q.s. | q.s. | q.s. | q.s. | q.s. | - |
| Benzophenon-4 | 0,2 | 0,25 | - | 0,3 | - | - | - |
| Oryzanol | - | - | - | 0,5 | - | - | 0,05 |
| Piroctone Olamine | - | - | - | - | - | 0.1 | 0.2 |
| Niacinamid | - | 0.01 | - | - | - | - | - |
| Panthenol | - | - | 0.01 | - | - | - | 0.01 |
| Jojobaöl | - | - | - | - | 0,1 | - | - |
| Natronlauge | q.s. | q.s. | q.s. | q.s. | q.s. | q.s. | - |
| Citronensäure | q.s. | q.s. | q.s. | q.s. | q.s. | q.s. | q.s. |
| Wasser | ad 100 | ad 100 | ad 100 | ad 100 | ad 100 | ad 100 | ad 100 |

### 2) Conditioner-Shampoo mit Perlglanz (und Trübungsmittel Formel 5)

| | **1** | **2** | **3** | **4** | **5** | **6** |
|---|---|---|---|---|---|---|
| Natrium Laurylethersulfat | 9 | 9 | 9 | 9 | 9 | 9 |
| Cocamidopropyl Betain | 4 | 3 | 4 | 3 | 4 | 3 |
| Dinatrium PEG-5 Laurylcitrat Sulfosuccinat | 3 | 3 | 3 | 2 | 3 | 4 |
| PEG-200 Hydrogenated Glyceryl Palmate | - | - | 0.1 | 0.2 | - | 0,4 |
| Guar Hydroxypropyltrimonium Chloride | 0.3 | 0.1 | 0.1 | 0.1 | 0.2 | 0,1 |
| Polyquaternium-7 | 0,1 | 0.2 | 0,3 | 0,4 | 0.2 | 0,5 |
| PEG-3 Distearat | 1.5 | 3 | 4 | 2 | 2,5 | 0,75 |
| Glycol Distearat | - | - | - | - | 0.5 | 0,5 |
| PEG-40 hydriertes Rizinusöl | 0.2 | 0.3 | 0.4 | 0.5 | 0.2 | 0,3 |
| PEG-90M | - | - | 0,05 | - | 0,1 | - |
| Natrium Salicylat | 0.2 | 0.2 | 0.4 | 0.4 | 0.4 | 0,2 |
| Natrium Benzoat | 0.4 | 0.4 | 0.4 | 0.4 | 0.4 | 0,4 |
| Natriumchlorid | 0,9 | 1,0 | 1,2 | 1,5 | - | 1,5 |
| Zitronensäure | q.s. | q.s. | q.s. | q.s. | q.s. | q.s. |
| Parfüm | q.s. | q.s. | q.s. | q.s. | q.s. | q.s. |
| Wasser | ad 100 | ad 100 | ad 100 | ad 100 | ad 100 | ad 100 |

### 3) Klares Conditioning-Shampoo

| | **1** | **2** | **3** | **4** | **5** | **6** |
|---|---|---|---|---|---|---|
| Natrium Laurylethersulfat | 10 | 9 | 3.5 | 3.5 | 0.5 | 8 |
| Natrium Myrethsulfat | - | - | 3.5 | 3.5 | 3.0 | 2 |
| Cocamidopropyl Betain | 4 | 4.5 | 3 | - | - | 3 |
| Natrium Cocoamphoacetat | - | - | - | 2.5 | - | 3 |
| Dinatrium PEG-5 Laurylcitrat Sulfosuccinat | - | - | - | - | 2.5 | 3 |
| Decylglucosid | - | - | - | 2 | 4.5 | - |
| PEG-200 Hydrogenated Glyceryl Palmate | 0.1 | - | - | 0.4 | 0.5 | - |
| Polyquaternium-7 | 0.1 | 0.1 | 0.2 | 0.3 | 0.4 | 0,5 |
| Guar Hydroxypropyltrimonium Chloride | 0,2 | 0.3 | 0,1 | 0,1 | 0.2 | 0,2 |
| Hydrolysiertes Seidenprotein | - | 0,1 | 0,2 | 0,3 | 0.3 | - |
| PEG-40 hydriertes Rizinusöl | 0.1 | 0.2 | 0.3 | 0.1 | 0.2 | 0,4 |
| Natrium Salicylat | - | - | 0.4 | - | | 0,2 |
| Natrium Benzoat | 0.5 | 0.5 | 0.4 | 0.4 | 0.4 | 0,4 |
| Benzophenon-4 | - | - | 0.1 | 0,2 | 0,3 | 0,4 |
| Zitronensäure | q.s. | q.s. | q.s. | q.s. | q.s. | q.s. |
| Parfüm | q.s. | q.s. | q.s. | q.s. | q.s. | q.s. |
| Wasser | ad 100 | ad 100 | ad 100 | ad 100 | ad 100 | ad 100 |

### 4) Mildes Baby Shampoo

| | **1** | **2** | **3** | **4** | **5** | **6** |
|---|---|---|---|---|---|---|
| Natrium Myristylethersulfat | 4 | 4 | 5 | 5 | 4 | 4 |
| Decylglucosid | 4 | 4 | 4 | 4 | 4 | 2 |
| Dinatrium PEG-5 Laurylcitrat Sulfosuccinat | 4 | 4 | 3 | 5 | 5 | 3 |
| PEG-80 Sorbitan Laurat | 2 | 1 | 1 | - | 0.5 | - |
| PEG-200 Hydrogenated Glyceryl Palmate | - | - | - | 0.2 | 0.3 | 0,4 |
| Polyquaternium-7 | 0.3 | 0.1 | 0.2 | 0.3 | 0,4 | 0,5 |
| Guar Hydroxypropyltrimonium Chloride | 0,2 | 0.3 | 0,1 | 0,1 | 0.2 | 0,2 |
| PEG-3 Distearat | - | - | 0,5 | 2 | - | 1 |
| Glycol Distearat | - | - | - | - | 0.5 | 0,5 |
| PEG-90M | - | - | 0,05 | - | 0,1 | - |
| PEG-40 hydriertes Rizinusöl | 0.2 | 0.3 | 0.4 | 0.2 | 0.3 | 0,4 |
| Natrium Salicylat | 0.2 | 0.2 | 0.2 | 0.4 | 0.4 | 0,4 |
| Natrium Benzoat | 0.4 | 0.4 | 0.4 | 0.4 | 0.4 | 0,4 |
| Natriumchlorid | 0.9 | 1.0 | 1.2 | 1,5 | 1,8 | - |
| Zitronensäure | q.s. | q.s. | q.s. | q.s. | q.s. | q.s. |
| Parfüm | q.s. | q.s. | q.s. | q.s. | q.s. | q.s. |
| Wasser | ad 100 | ad 100 | ad 100 | ad 100 | ad 100 | ad 100 |

### 5) Antischuppen Shampoo/Mildes Kopfhaut Shampoo (Formel 6)

| | **1** | **2** | **3** | **4** | **5** | **6** |
|---|---|---|---|---|---|---|
| Natrium Laurylethersulfat | 9 | 9 | 9 | 9 | 10 | - |
| Natrium Myristylethersulfat | - | - | - | - | - | 6 |
| Cocamidopropyl Betain | 4 | 4 | 3 | 3 | 4 | 5 |
| Dinatrium PEG-5 Laurylcitrat Sulfosuccinat | 3 | 3 | 3 | 3 | - | - |
| Natrium Cocoamphoacetat | - | - | - | - | - | 2.5 |
| Decylglucosid | - | - | - | - | - | 2.5 |
| PEG-200 Hydrogenated Glyceryl Palmate | - | - | 0.1 | - | 0.4 | 3 |
| Polyquaternium-7 | 0.3 | 0.1 | 02 | 0.3 | 0.1 | 0.5 |
| Guar Hydroxypropyltrimonium Chloride | 0,2 | 0,3 | 0.2 | 0.1 | 0,2 | 0,1 |
| Climbazol | 0.5 | 0.5 | - | 0.5 | 1.0 | - |
| Piroctone Olamin | - | 0.5 | 0.3 | - | 0.5 | - |
| Laureth-9 | - | - | - | - | 2 | 2 |
| Panthenol | - | - | - | - | - | 0,1 |
| Harnstoff | | | | 3 | 4 | 5 |
| PEG-3 Distearat | 1.5 | 3 | 0,75 | 0,5 | - | - |
| Glycol Distearat | - | - | - | - | 0.5 | - |
| PEG-40 hydriertes Rizinusöl | 0.2 | 0.3 | 0.4 | 0.5 | 0.2 | - |
| Natrium Salicylat | 0.4 | 0.4 | 0.4 | 0.4 | 0.4 | 0.2 |
| Natrium Benzoat | 0.4 | 0.4 | 0.4 | 0.4 | 0.4 | 0.4 |
| Natriumchlorid | 0.9 | 1.0 | 1.2 | - | - | - |
| Zitronensäure | q.s. | q.s. | q.s. | q.s. | q.s. | - |
| Milchsäure | - | - | - | - | - | q.s. |
| Parfüm | q.s. | q.s. | q.s. | q.s. | q.s. | q.s |
| Wasser | ad 100 | ad 100 | ad 100 | ad 100 | ad 100 | ad 100 |

## Patentansprüche

1. Kosmetische und dermatologische waschaktive Zubereitungen zur Reinigung der Haut und/oder zur Haar- und Kopfhautreinigung, enthaltend Dimethyldiallylammoniumchlorid-Acrylamid Copolymere, kationisch modifizierte Guarderivate, Alkylethersulfate und Cocamidopropylbetain.

2. Zubereitung nach Patentanspruch 1 zusätzlich enthaltend Alkylpolyglykolestersulfosuccinate.

3. Zubereitungen nach einem der vorangehenden Patentansprüche , **dadurch gekennzeichnet, dass** Dimethyldiallylammoniumchlorid-Acrylamid Copolymer mit einem Molekulargewicht von 1,0x10⁶ g/mol bis 3x10⁶ g/mol und einer Ladungsdichte von 1,0 bis 3,5 meg/g verwendet wird, bevorzugt mit einem Molekulargewicht von 1,4x10⁶ g/mol bis 1,8x10⁶ g/mol und einer Ladungsdichte von 2,5 bis 3,5 meg/g, besonders bevorzugt mit einem Molekulargewicht von 1,6x10⁶ g/mol und einer Ladungsdichte von 3,1 meg/g.

4. Zubereitungen nach einem der vorhergehenden Ansprüche, **dadurch gekennzeichnet, dass** als kationisch modifizierte Guarderivate solche mit einem Molekulargewicht von 50.000 bis 2.500.000, besonders bevorzugt von 1.000.000 bis 1.500.000 verwendet werden.

5. Zubereitungen nach einem der vorhergehenden Ansprüche, **dadurch gekennzeichnet, dass** als kationisch modifizierte Guarderivate solche mit einem Molekulargewicht von 1.300000 g/mol und einer mittleren Ladungsdichte von 0.4-0.6 meq/g verwendet werden.

6. Zubereitungen nach einem der vorhergehenden Ansprüche, **dadurch gekennzeichnet, dass** als kationisch modifiziertes Guarderivat Guar Hydroxypropyltrimonium Chlorid verwendet wird.

7. Zubereitungen nach einem der vorhergehenden Ansprüche, **dadurch gekennzeichnet, dass** die Einsatzkonzentrationen an Dimethyldiallylammoniumchlorid-Acrylamid Copolymer und kationisch modifizierten Guarderivaten jeweils 0,01 bis 1 Gew.%., besonders bevorzugt 0,01 bis 0,5 Gew.%.betragen.

8. Zubereitungen nach einem der vorhergehenden Ansprüche, **dadurch gekennzeichnet, dass** mindestens ein Di-Natrium Salz eines Alkylpolyglycolestersulfosuccinats der Äpfel-, Milch- oder Citronensäure enthalten ist.

9. Zubereitungen nach einem der vorhergehenden Ansprüche, **dadurch gekennzeichnet, dass** als Tenside anionische, amphotere und nichtionische Tenside in Gesamtkonzentrationen von 2 bis 25 Gew.-%. verwendet werden.

10. Zubereitungen nach einem der vorhergehenden Ansprüche, **dadurch gekennzeichnet, dass** als Tenside die Kombination von Laurylethersulfat mit Cocamidopropylbetain verwendet wird.

11. Zubereitungen nach einem der vorhergehenden Ansprüche, **dadurch gekennzeichnet, dass** zusätzlich PEG-3 Distearat mit Teilchengrößen verwendet wird, die so bemessen sind, dass 90 % der Volumenanteile eine Teilchengröße von kleiner 50 µm aufweisen.
